# EUROPEAN PATENT APPLICATION

(11) **EP 4 672 258 A1**
(43) Date of publication of application: **31.12.2025**
(21) Application number: 25180113.0
(22) Date of filing: 02.06.2025
(51) Int. Cl.: G16H 40/63, A61B 8/00

(54) **SYSTEM AND METHOD FOR INTEGRATING ULTRASOUND IMAGING WITH AN ANESTHESIA SYSTEM**

(30) Priority: 25.06.2024 US 202418753270
(71) Applicant: GE Precision Healthcare LLC, Waukesha, WI 53188 (US)
(72) Inventor: JEGATHALA RAJU, Nandakumar, 560066 Bangalore (IN); CHRISTOPHER SELVI, Godwin, 560066 Bangalore (IN); HÄGGBLOM, Tom Jakob, Waukesha, 53188 (US); KOTTADAMANE ASWATHNARAYAN, Sharath, 560066 Bangalore (IN); RAVINUTHALA, Ramakrishna Rao, 560066 Bangalore (IN); MALAGI, Harsha, 560066 Bangalore (IN)
(74) Representative: Kilburn & Strode LLP

(57) **Abstract**

An anesthesia system includes a housing, a display system coupled to housing, and an ultrasound probe communicatively coupled to the anesthesia system. The anesthesia system includes a memory encoding processor-executable routines disposed within the housing. The anesthesia system includes a processing system disposed within the housing. The processing system includes one or more processors and is configured to access the memory and to execute the processor-executable routines, wherein the processor-executable routines, when executed by the processing system, cause the processing system to perform actions. The actions include receiving scan data of a subject receiving anesthesia therapy from the ultrasound probe. The actions also include receiving patient vital data representative of anesthesia monitoring parameters of the subject from sources of patient vital data or sensors. The actions further include displaying the anesthesia monitoring parameters and an ultrasound image derived from the scan data on the display system.

## Description

### BACKGROUND

The subject matter disclosed herein relates to a system and a method for integrating ultrasound imaging with an anesthesia system.

Currently, based on the patient's condition, an anesthesiologist may request an ultrasound imaging system to be made available at an operation theater. The operation theater will already have a few monitoring devices within the vicinity of the patient. The presence of one or more additional devices will make the process cumbersome. In many cases, an anesthesia system and an ultrasound unit are placed on opposite sides of the clinician, thereby requiring the clinician to turn his attention away from either the surgical procedure or the anesthesia system to view the ultrasound display. One of the significant challenges of utilizing separate anesthesia systems and ultrasound systems is time-based synchronization of the two displays when reviewing the stored information. The stored images form the ultrasound unit and the hemodynamic measurements from the anesthesia system are not integrated with each other. Thus, the time-based information is not correlated or synchronized to the other unit.

### BRIEF DESCRIPTION

Certain embodiments commensurate in scope with the originally claimed subject matter are summarized below. These embodiments are not intended to limit the scope of the claimed subject matter, but rather these embodiments are intended only to provide a brief summary of possible embodiments. Indeed, the invention may encompass a variety of forms that may be similar to or different from the embodiments set forth below.

In one embodiment, an anesthesia system is provided. The anesthesia system includes a housing. The anesthesia system also includes a display system coupled to housing. The anesthesia system further includes an ultrasound probe communicatively coupled to the anesthesia system. The anesthesia system even further includes a memory encoding processor-executable routines disposed within the housing. The anesthesia system still further includes a processing system disposed within the housing. The processing system includes one or more processors and is configured to access the memory and to execute the processor-executable routines, wherein the processor-executable routines, when executed by the processing system, cause the processing system to perform actions. The actions include receiving scan data of a subject receiving anesthesia therapy from the ultrasound probe. The actions also include receiving patient vital data representative of one or more anesthesia monitoring parameters of the subject from sources of patient vital data or sensors. The actions further include displaying the one or more anesthesia monitoring parameters, anesthesia therapy settings and an ultrasound image derived from the scan data on the display system.

In another embodiment, a computer-implemented method for integrating ultrasound imaging with an anesthesia system is provided. The computer-implemented method includes receiving, at a processing system including one or more processors disposed within a housing of the anesthesia system, scan data of a subject from the ultrasound probe, wherein the subject is receiving anesthesia therapy, and the ultrasound probe is communicatively coupled to the anesthesia system. The computer-implemented method also includes receiving, at the processing system, d patient vital data representative of one or more anesthesia monitoring parameters of the subject from sources of patient vital data or sensors. The computer-implemented method further includes displaying, via the processing system, the one or more anesthesia monitoring parameters and an ultrasound image derived from the scan data on a display system coupled to the housing.

In a further embodiment, a non-transitory computer-readable medium is provided. The computer-readable medium includes processor-executable code that when executed a processing system including one or more processors disposed within a housing of an anesthesia system causes the processing system to perform actions. The actions include receiving scan data of a subject from an ultrasound probe, wherein the subject is receiving anesthesia therapy, and the ultrasound probe is communicatively coupled to the anesthesia system. The actions also include receiving d patient vital data representative of one or more anesthesia monitoring parameters of the subject from /sources of patient vital data or sensors. The actions further include simultaneously displaying the one or more anesthesia monitoring parameters, anesthesia therapy settings, and an ultrasound image derived from the scan data on a display of a display system coupled to the housing.

### BRIEF DESCRIPTION OF THE DRAWINGS

These and other features, aspects, and advantages of the present subject matter will become better understood when the following detailed description is read with reference to the accompanying drawings in which like characters represent like parts throughout the drawings, wherein:
FIG. 1 is a schematic diagram of an integrated system, in accordance with aspects of the present disclosure;
FIG. 2 is a schematic diagram of the integrated system in FIG. 1 functioning as a gateway for ultrasound imaging, in accordance with aspects of the present disclosure;
FIG. 3 is a schematic diagram of an anesthesia system functioning as a gateway for ultrasound imaging (e.g., for a separate ultrasound imaging device), in accordance with aspects of the present disclosure;
FIG. 4 is a schematic diagram of a separate anesthesia system and a separate ultrasound imaging device communicating with a cloud-based system via a separate gateway, in accordance with aspects of the present disclosure;
FIG. 5 is a schematic diagram of a separate anesthesia system and a separate ultrasound imaging device communicating with a cloud-based system via a separate gateway (e.g., utilizing highspeed low latency networks), in accordance with aspects of the present disclosure;
FIG. 6 is a schematic diagram of a separate anesthesia system and a separate ultrasound imaging device communicating directly with a cloud-based system (e.g., utilizing highspeed low latency networks), in accordance with aspects of the present disclosure;
FIG. 7 is a schematic diagram of a separate anesthesia system and a separate ultrasound imaging device communicating directly with each other (e.g., utilizing a highspeed low latency network), in accordance with aspects of the present disclosure;
FIG. 8 is a schematic diagram of an ultrasound probe communicatively coupled via a wireless connection to an anesthesia system, in accordance with aspects of the present disclosure;
FIG. 9 is a schematic diagram of an ultrasound probe communicatively coupled to a separate device communicatively coupled to an anesthesia system, in accordance with aspects of the present disclosure;
FIG. 10 is a schematic diagram of an ultrasound probe communicatively coupled to a separate device communicatively coupled to an anesthesia system (which is communicatively coupled to a cloud-based system), in accordance with aspects of the present disclosure;
FIG. 11 is a schematic diagram of an anesthesia system configured to display ultrasound data, anesthesia monitoring parameter data and anesthesia therapy settings (e.g., having a single display), in accordance with aspects of the present disclosure;
FIG. 12 is a schematic diagram of an anesthesia system configured to display ultrasound data, anesthesia monitoring parameter data and anesthesia therapy settings (e.g., having a pair of displays), in accordance with aspects of the present disclosure;
FIG. 13 is a schematic diagram of an anesthesia system configured to display ultrasound data, anesthesia monitoring parameter data and anesthesia therapy settings (e.g., also having a pair of displays in a different arrangement), in accordance with aspects of the present disclosure; and
FIG. 14 is a schematic diagram of an anesthesia system configured to display ultrasound data, anesthesia monitoring parameter data and anesthesia therapy settings (e.g., having a plurality of displays), in accordance with aspects of the present disclosure.

### DETAILED DESCRIPTION

One or more specific embodiments will be described below. In an effort to provide a concise description of these embodiments, not all features of an actual implementation are described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present subject matter, the articles "a," "an," "the," and "said" are intended to mean that there are one or more of the elements. The terms "comprising," "including," and "having" are intended to be inclusive and mean that there may be additional elements other than the listed elements. Furthermore, any numerical examples in the following discussion are intended to be nonlimiting, and thus additional numerical values, ranges, and percentages are within the scope of the disclosed embodiments.

As may be appreciated, implementations of the present disclosure may be embodied as a system, method, device, or computer program product. Accordingly, aspects of the present disclosure may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present disclosure may take the form of a computer program product embodied in one or more computer readable medium(s) having computer-readable program code embodied thereon.

The present disclosure provides systems and methods for integrating ultrasound imaging with an anesthesia system (e.g., anesthesia system). The disclosed embodiments includes a single device having functionalities of both the anesthesia system and the ultrasound imaging system. In certain embodiments, the single device may include a display system with multiple displays (e.g., monitors) that enable the viewing of the ultrasound imaging data, one or more measured anesthesia monitoring parameters of a subject (e.g., patient) undergoing anesthesia therapy, anesthesia therapy settings, and/or entertainment (e.g., music, movies, etc.) for the subject. The disclosed embodiments enable the anesthesiologist/surgeon to monitor the data side by side. The disclosed embodiments provide the synchronization of the data to help the anesthesiologist/surgeon make decisions for the subject. Based on the clinical need, in certain embodiments, the same display can be used either completely for viewing/monitoring anesthesia parameters and anesthesia therapy settings or for ultrasound imaging/measurements.

The disclosed embodiments include an anesthesia system (e.g. integrated system) including a housing, a display system coupled to housing, and an ultrasound probe communicatively coupled to the anesthesia system. In certain embodiments, the ultrasound probe is communicatively coupled to the anesthesia system via a wireless connection. In certain embodiments, the ultrasound probe is communicatively coupled to the anesthesia system via a wired connection (e.g., via connector on the housing that enables plug and play capability with different ultrasound probes). The anesthesia system even further includes a memory encoding processor-executable routines disposed within the housing. The anesthesia system still further includes a processing system disposed within the housing. The processing system includes one or more processors and is configured to access the memory and to execute the processor-executable routines, wherein the processor-executable routines, when executed by the processing system, cause the processing system to perform actions. The actions include receiving scan data of a subject receiving anesthesia therapy from the ultrasound probe. The actions also include receiving patient vital data representative of one or more anesthesia monitoring parameters of the subject from , the sources of patient vital (e.g., a patient monitor or a patient vital acquisition system or from the patient vital sensors or a combination etc.). The actions further include displaying the one or more anesthesia monitoring parameters, anesthesia therapy settings and an ultrasound image derived from the scan data on the display system.

In certain embodiments, the display system includes a display and the one or more anesthesia monitoring parameters, anesthesia therapy settings and the ultrasound image are simultaneously displayed on the display. In certain embodiments, the display system includes a plurality of displays, and the one or more anesthesia monitoring parameters, anesthesia therapy settings and the ultrasound image are simultaneously displayed on separate displays of the plurality of displays. In certain embodiments, the actions include displaying, via the processing system, anesthesia therapy settings on the display system. In certain embodiments, the display system includes a display and the one or more anesthesia monitoring parameters, anesthesia therapy settings the ultrasound image, and the anesthesia therapy settings are simultaneously displayed on the display. In certain embodiments, the one or more anesthesia monitoring parameters, anesthesia therapy settings and the ultrasound image are displayed in real time on the display system.

In certain embodiments, the actions include storing, via the processing system, the scan data and the patient vital data in the memory. In certain embodiments, the actions include providing, via the processing system, the scan data and the patient vital data to a cloud-based system for processing and analysis and receiving, via the processing system, the ultrasound image, the one or more anesthesia monitoring parameters, anesthesia therapy settings and the analysis of the scan data and the patient vital data from the cloud-based system. In certain embodiments, the actions include providing, via the processing system, the scan data and the patient vital data to a cloud-based system for storage.

In certain embodiments, the actions include providing the scan data and the patient vital data to a cloud-based system for synchronization of the data prior to display of the one or more anesthesia monitoring parameters, anesthesia therapy settings and the ultrasound image. In certain embodiments, the actions include synchronizing, via the processing system, the scan data and the patient vital data. In certain embodiments, the actions include providing, via the processing system, the scan data and the patient vital data to one or more of a hospital network, a picture archiving and communication system, an electronics medical records system, or a cloud-based storage system.

The disclosed embodiments enable more effective use of operating room space. The disclosed embodiments also include reducing the stress on the clinician and anesthesiologist by providing ultrasound data and anesthesia data on a single display. The disclosed embodiments further include providing ultrasound imaging capability in an anesthesia system will aid the anesthesiologist in making more accurate predictions and produce better outcomes for the patient. The disclosed embodiments make the machine (e.g., anesthesia system) safer, more accurate, and faster.

The benefits of the disclosed embodiments include, if the anesthesiologist determines that a patient's heart condition is weak or at risk, the clinician will monitor the heart using transesophageal echocardiography. The disclosed embodiments also provide benefits to ultrasound guided regional anesthesia. One of the principal challenges of regional anesthesia is the unreliability of conventional modalities for confirming precise nerve localization. A successful regional block requires optimal distribution of local anesthetic around nerve and plexus structures. Real time ultrasound guidance serves as an aid in nerve localization. The disclosed embodiments also enable ultrasound for vascular access. The disclosed embodiments also enable airway assessment. Ultrasound imaging of the upper airway provides another objective modality to anesthesiologists to assess the size of the airway, the presence of foreign bodies, and the condition and structure of the epiglottis. The ability to measure airway dimensions is important for clinicians and interventional bronchoscopists to accurately quantify structural abnormalities in response to treatment. The disclosed embodiments also enable lung ultrasound. The disclosed embodiments also enable assessment of the abdomen to determine residual urine volumes and aid suprapubic catheterization. The disclosed embodiments enable assessment of the urinary tract obstruction or catheter patency. The disclosed embodiments are also beneficial for a central neuraxial block. Spinal anesthesia can be challenging in patients with poorly palpable surface landmarks and in age-related changes in the lumbar spine. In particular, the elderly and obese patients present technical difficulties due to poor quality surface landmarks and reduced ability to flex the lumbar spine. Ultrasound imaging has been shown to be superior to clinical palpitation as a technique for identifying lumbar intervertebral level.

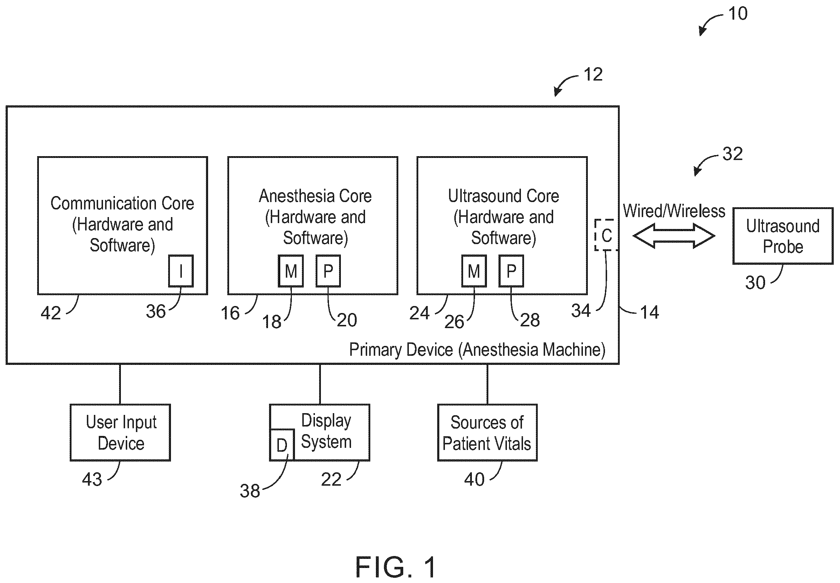

FIG. 1 is a schematic diagram of an integrated system 10 (e.g., combined anesthesia system and ultrasound imaging system). As depicted, the integrated system 10 provides a single device that integrates ultrasound imaging into an anesthesia system 12. The anesthesia system 12 serves as the primary device for the integrated system 10. The integrated system 10 has the advantage of providing less equipment in an operating room, thus, saving space. The anesthesia system 12 is configured to monitor a status of a patient undergoing anesthesia therapy (e.g., during a surgical procedure). In particular, the anesthesia system 12 is configured to monitor a number of parameters (e.g., measured parameters) of the patient, some which are related to the application of the anesthesia therapy and anesthesia monitoring . These parameters may include ,blood pressure, heart rate, oxygen saturation, electrical heart activity (e.g., electrocardiogram data) or heart waveforms, and other parameters.

The integrated system 10 includes a housing 14. The integrated system 10 also includes an anesthesia core 16 (e.g., hardware and software) disposed within the housing 14. The anesthesia core 16 includes a memory 18 and a processing system 20. The processing system 20 may include one or more processors. The processing system 20 executes instructions contained in the memory 18. Execution of the instructions causes the processing system 20 to perform steps related to the operation of the anesthesia system 12. The instructions may be loaded in a random-access memory (RAM) for execution by the processing unit from a read only memory (ROM), a mass storage device, or some other persistent storage. In other embodiments, hard wired circuitry may be used in place of or in combination with software instructions to implement the functions described. For example, the processing system 20 may be embodied as part of one or more application-specific integrated circuits (ASICs). Unless otherwise specifically noted, the anesthesia core 16 is not limited to any specific combination of hardware circuitry and software, nor to any particular source for the instructions executed by the processing unit.

The anesthesia core 16 is configured to monitor the patient during anesthesia by receiving, processing, and/to analyze the data measured of the patient. In addition, the anesthesia core 16 is configured to monitor the parameters for providing the anesthesia therapy. The anesthesia core 16 is also configured to enable the adjustment of the anesthesia settings for applying the anesthesia therapy. The anesthesia core 16 is also configured to cause display of measured parameters of the patient subject to anesthesia therapy, anesthesia parameters, and/or anesthesia settings (e.g., on a display system 22 of the anesthesia system 12).

The integrated system 10 also includes an ultrasound core 24 (e.g., hardware and software) disposed within the housing 14. The ultrasound core 24 includes a memory 26 and a processing system 28. The processing system 28 may include one or more processors. The processing system 28 executes instructions contained in the memory 26. Execution of the instructions causes the processing system 28 to perform steps related to the operation of ultrasound imaging. The instructions may be loaded in a random-access memory (RAM) for execution by the processing unit from a read only memory (ROM), a mass storage device, or some other persistent storage. In other embodiments, hard wired circuitry may be used in place of or in combination with software instructions to implement the functions described. For example, the processing system 28 may be embodied as part of one or more application-specific integrated circuits (ASICs). Unless otherwise specifically noted, the ultrasound core 24 is not limited to any specific combination of hardware circuitry and software, nor to any particular source for the instructions executed by the processing unit.

The ultrasound core 24 is configured to receive scan data from an ultrasound probe 30 that is communicatively coupled to the anesthesia system 12. The ultrasound probe 30 and the ultrasound core 24 form an ultrasound imaging system 32 integrated within the anesthesia system 12. The ultrasound core 24 is configured to receive scan data from the ultrasound probe 30. In certain embodiments, the ultrasound core 24 is configured to process the scan data to generate ultrasound images (e.g., of a subject undergoing anesthesia therapy). In certain embodiments, the ultrasound core 24 is configured to determine and provide ultrasound measurements (e.g., in response to input from a user). In certain embodiments, the ultrasound core 24 is configured to cause display of ultrasound images and/or ultrasound measurements on the display system 22.

In certain embodiments, the ultrasound probe 30 is communicatively coupled to the anesthesia system 12 via a wired connection. For example, one or more connectors may be disposed on the housing 14 to enable a connector of a wire coupled to the ultrasound probe 30 to plug into. The connectors 34 of the anesthesia system 12 enables the coupling of different ultrasound probes in a plug and play manner. In certain embodiments, the ultrasound probe 30 (e.g., wireless ultrasound probe such as Vscan Air^{™} from GE Healthcare) is communicatively coupled to the anesthesia system 12 via a wireless connection. In certain embodiments, the ultrasound probe 30 may communicate with a wireless communication interface 36 within the anesthesia system 12 (e.g., via Wi-Fi Direct and Bluetooth).

In certain embodiments, instead of having a separate anesthesia core 16 and a separate ultrasound core 24, the anesthesia system 12 may include a common or single core. The common core may be configured to perform the functionalities of both the anesthesia core 16 and the ultrasound core 24. The common core also configured to receive and process data from the sources of patient vital data 40 (e.g., a patient monitor or a patient vital acquisition system or from the patient vital sensors or a combination etc.). In certain embodiments, the anesthesia core 16, the ultrasound core 24, and/or the common core is configured to synchronize (and time stamp) the data from anesthesia parameter monitoring, anesthesia therapy settings and ultrasound imaging. In certain embodiments, the anesthesia core 16, the ultrasound core 24, and/or the common core is configured also to simultaneously display the measured parameters of the patient subject to anesthesia therapy, anesthesia parameters monitoring, anesthesia therapy settings, and ultrasound images and/or ultrasound measurements on the display system 22. In certain embodiments, the anesthesia core 16, the ultrasound core 24, and/or the common core is configured to enable the measured parameters of the patient subject to anesthesia therapy, anesthesia parameters monitoring, anesthesia therapy settings, and ultrasound images and/or ultrasound measurements to be displayed in real time with the lowest possible latency. In certain embodiments, the anesthesia core 16, the ultrasound core 24, and/or the common core is configured to store the measured parameters of the patient subject to anesthesia therapy, anesthesia parameters monitoring, anesthesia therapy settings, and ultrasound images and/or ultrasound measurements on the anesthesia monitoring system 12.

The anesthesia system 12 also includes the display system 22 for displaying measured parameters of the patient subject to anesthesia therapy, anesthesia parameters monitoring, anesthesia therapy settings, and ultrasound images and/or ultrasound measurements. The display system 22 is coupled to the housing 14. In certain embodiments, the display system 22 includes a single display 38. In certain embodiments, the display system 22 includes a plurality of displays 38 (e.g., 2, 3, 4, or more displays 38). In certain embodiments, the anesthesia therapy settings, anesthesia parameters monitoring, and ultrasound images and/or ultrasound measurements are displayed on respective displays 38. In certain embodiments, a combination of at least two of the anesthesia therapy settings, anesthesia parameters monitoring, and ultrasound images and/or ultrasound measurements are displayed on respective displays 38. In certain embodiments, anesthesia therapy settings, anesthesia parameters monitoring, and ultrasound images and ultrasound measurements are displayed on the same display 38. In certain embodiments, entertainment (e.g., videos, movies, etc.) may be displayed on one of displays 38 to entertain the patient / to keep the patient (specially kids) occupied as a method of diversion from the ongoing clinical procedure.

The anesthesia system 12 is communicatively coupled (via wired communication and/or wireless communication) to sources of patient vital data 40 (e.g., a patient monitor or a patient vital acquisition system or from the patient vital sensors or a combination etc.). In certain embodiments, the sources of patient vital data 40 are disposed on or coupled to the patient to measure parameters (e.g., anesthesia parameters, hemodynamic parameters, etc.) of the subject. These parameters may include blood pressure, heart rate, oxygen saturation, electrical heart activity (e.g., electrocardiogram data) or heart waveforms, and other parameters. In certain embodiments, the sources of patient vital data 40 may be associated with components of a machine providing the anesthesia therapy and/or a ventilator and measure parameters related to the operation of these machines (e.g., inspiration, expiration, amount of various gases being administered, etc.).

The integrated system 10 also includes a communication core 42 (e.g., hardware and software) disposed within the housing 14. The communication core 42 provides a single communication point for communicating both the ultrasound data and anesthesia data to another destination (e.g., one or more of a hospital network, a picture archiving and communication system (PACS), an electronics medical records (EMR) system, or a cloud-based storage/analysis system). The communication core 42 includes one or more communication interfaces 36 for communicating via a wired or wireless communication utilizing one or more communication protocols. In certain embodiments, one of the communication interfaces 36 is configured to wirelessly pair (e.g., via Bluetooth^{®}) with the wireless ultrasound probe 30 and to exchange date (e.g., Wi-Fi Direct) with the wireless ultrasound probe. In certain embodiments, the communication core 42 may communicate via local area networks (LANs) and/or wide-area networks (WANs) and/or serial protocols like RS232/422/485 and/or other standard communication protocols.

The anesthesia system 12 also includes a user input device 43. The user input device 43 may include one or more of a touchscreen, a keyboard, a mouse, a trackpad, a motion sensing camera, or other device configured to enable a user to interact with and manipulate data within anesthesia system 12. In one example, the user input device 43 may enable a user to make a selection as to what data should be displayed on which display 38 of the display system 22. In another example, the user input device 43 may enable a user to enlarge one set of data (e.g., ultrasound image) relative to another set of data (e.g., parameters of the patient) when displayed on the same display 38.

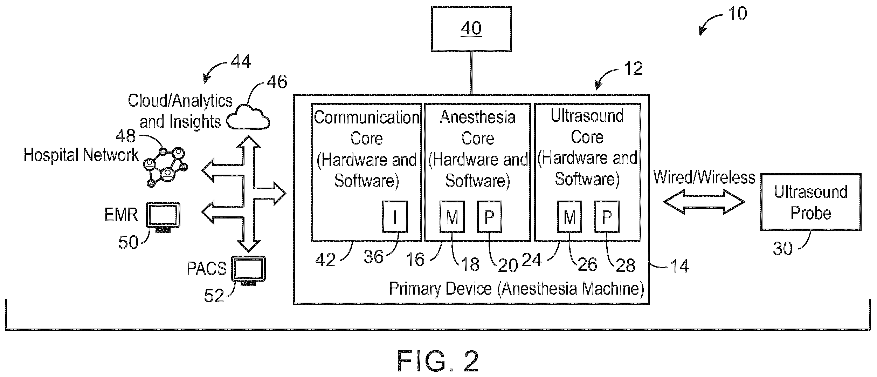

FIG. 2 is a schematic diagram of the integrated system 10 in FIG. 1 functioning as a gateway for ultrasound imaging. As depicted, the communication core 42 of the anesthesia system 12 is configured to act as a gateway (e.g., single communication point) to provide both the ultrasound imaging data (e.g., ultrasound scan data and/or ultrasound images) and the anesthesia data to one or more different systems/networks 44 separate from the integrated system 10. Sources of patient vital data 40 are connected to the anesthesia system 12. In certain embodiments, one or more of the different systems/networks 44 provide processed, analyzed, and/or time-synchronized data (e.g., ultrasound scan data and/or ultrasound images and anesthesia therapy settings and anesthesia monitoring parameter data) back to the integrated system 10. Providing the correlated ultrasound data, anesthesia therapy settings and anesthesia monitoring parameter data together across the network (via the communication core 42) is more useful than providing the data independently.

As depicted, the different systems/networks 44 include a cloud-based storage/analysis system 46, a hospital network 48, an EMR system 50, and PACS 52. Sharing data via a single communication connection (via the anesthesia system 12) to the EMR 50 enables record keeping which also tags patient data (e.g., patient history). Sending data to the cloud-base storage/analysis system 46 enables the analysis and persistence of the data as well as remote viewing of the data on other devices (e.g., smart tablets, smart phones, computers, consoles, etc.) different from the anesthesia system 12. Since the anesthesia system 12 acts as the gateway (e.g., bedside aggregator) to the hospital network 48, the options for communication are not limited to the cloud-based storage/analysis system 46, the EMR 50 and the PACS 52 but may be extended to any future application.

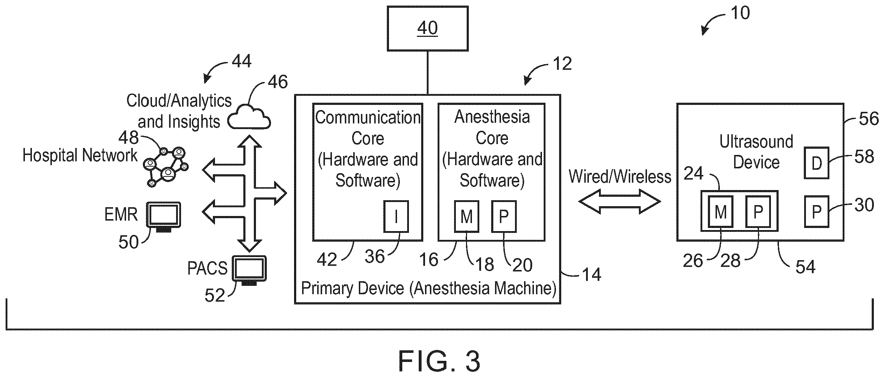

FIG. 3 is a schematic diagram of the anesthesia system 12 functioning as a gateway for ultrasound imaging (e.g., for a separate ultrasound imaging device 54). As depicted, the anesthesia system 12 is not part of an integrated system. Instead, the ultrasound imaging device 54 is separate from the anesthesia system 12. Sources of patient vital data 40 are connected to the anesthesia system 12. The anesthesia system 12 includes the anesthesia core 16 and the communication core (e.g., as described in FIG. 1) disposed within the housing 14. The separate ultrasound imaging device 54 includes the ultrasound core 24 (e.g., as described in FIG. 1) disposed within a housing 56. The ultrasound imaging device 54 also includes the ultrasound probe 30. In certain embodiments, the ultrasound core 24 may be disposed within the probe 30 itself. In certain embodiments, the ultrasound imaging device 54 includes its own display 58. In certain embodiments, the ultrasound imaging device 54 is communicatively coupled to the anesthesia system 12 (i.e., the communication core 42) via a wired connection. In certain embodiments, the ultrasound imaging device 54 is communicatively coupled to the anesthesia system 12 (i.e., the communication core 42) via a wireless connection.

As depicted, the communication core 42 of the anesthesia system 12 is configured to act as a gateway (e.g., single communication point) to provide the ultrasound imaging data (e.g., ultrasound scan data and/or ultrasound images), anesthesia therapy settings and the anesthesia monitoring parameters data to one or more different systems/networks 44 separate from both the anesthesia system 12 and the ultrasound imaging device 54. In certain embodiments, one or more of the different systems/networks 44 provide processed, analyzed, and/or time-synchronized data (e.g., ultrasound scan data and/or ultrasound images, anesthesia therapy settings and anesthesia monitoring parameters data) back to the anesthesia system 12 and/or the ultrasound imaging device 54 via the anesthesia device (e.g., via the communication core 42). Providing the correlated ultrasound data, anesthesia therapy settings and anesthesia monitoring parameters data across the network (via the communication core 42) is more useful than the providing the data independently.

Sharing data via a single communication connection (via the anesthesia system 12) to the EMR 50 enables record keeping which also tags patient data (e.g., patient history). Sending data to the cloud-base storage/analysis system 46 enables the analysis and persistence of the data as well as remote viewing of the data on other devices (e.g., smart tablets, smart phones, computers, consoles, etc.) different from the anesthesia system 12. Since the anesthesia system 12 acts as the gateway (e.g., bedside aggregator) to the hospital network 48, the options for communication are not limited to the cloud-based storage/analysis system 46, the EMR 50 and the PACS 52 but may be extended to any future application.

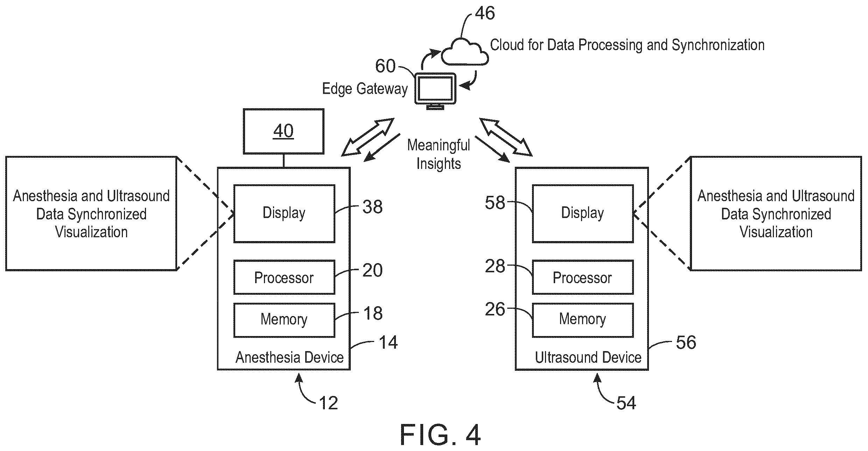

FIG. 4 is a schematic diagram of a separate anesthesia system 12 and a separate ultrasound imaging device 54 communicating with the cloud-based storage/analysis system 46 via a separate gateway 60. The anesthesia system 12 and the ultrasound imaging device 54 are as described in FIG. 3. However, instead of utilizing the anesthesia system 12 as a gateway, both the anesthesia system 12 and the ultrasound imaging device 54 separately communicate with the cloud-based storage/analysis system 46 via an independent gateway 60. In particular, the gateway 60 (e.g., network node) connects both the anesthesia system 12 and the ultrasound imaging device 54 with the cloud-based storage/analysis system 46 (e.g., where the cloud-based storage/analysis system 46 has a different communication protocol than a respective communication protocol of both the anesthesia monitoring system 12 and the ultrasound imaging device 54). Sources of patient vital data 40 are connected to the anesthesia system 12. In certain embodiments, the gateway 60 is an edge gateway or internet of things (IoT) gateway that couples IoT devices to edge infrastructure (e.g., the cloud). The anesthesia device 12 communicates the anesthesia therapy settings and anesthesia monitoring parameters data (e.g., parameter measurements of the patient) to the cloud-based storage/analysis system 46 via the gateway 60. The ultrasound imaging device 54 communicates the scan data and/or ultrasound images and/or patient vital to the cloud-based storage/analysis system 46 via the gateway 60. Sources of patient vital data 40 communicates the patient vital data to the cloud based storage/analysis system 46 via the gateway 60. In particular, the data gathered by the anesthesia system 12, the sources of patient vital data 40, and the ultrasound imaging device 54 is provided to the cloud-based storage/analysis system 46 via the gateway. In certain embodiments, the cloud-based storage/analysis system 46 is configured to process (e.g., generate ultrasound image), to analyze, and to synchronize (e.g., add time stamps) the patient vital data and the scan data. The processed data (which may be delayed but is synchronized) and/or meaningful insights from the analysis is relayed from the cloud-based storage/analysis system 46 back to both the anesthesia system 12 and the ultrasound imaging device 54 for simultaneous visualization on the display 38 of the anesthesia system 12, the display 58 of the ultrasound imaging device 54, or on both the display 38 of the anesthesia system 12 and the display 58 of the ultrasound imaging device 54. In certain embodiments, a combination of at least a portion of the anesthesia-related data (e.g., anesthesia or hemodynamic parameters) and the ultrasound data (e.g., ultrasound images and/or ultrasound measurements) are displayed simultaneously on the display 38, the display 58, or both the displays 38, 58. The embodiment in FIG. 4 helps with the visualization of the data, thus, making it more comfortable for the clinician to use either of the anesthesia system 12 and the ultrasound imaging device 54 for visualization and command and control. In certain embodiments, the ultrasound data, anesthesia therapy settings and the anesthesia monitoring parameters data may be stored on the cloud-based storage/analysis system 46. In certain embodiments, the ultrasound data, anesthesia therapy settings and the anesthesia monitoring parameters data may be stored on the anesthesia system 12.

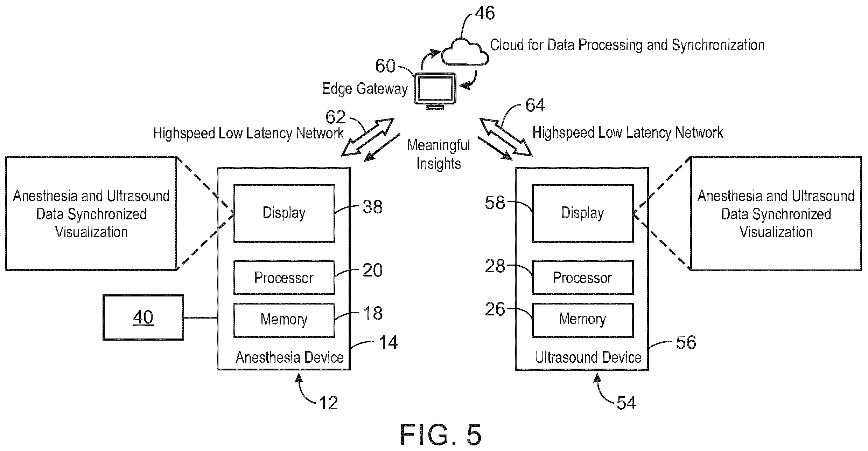

FIG. 5 is a schematic diagram of the separate anesthesia system 12 and the separate ultrasound imaging device 54 communicating with the cloud-based storage/analysis system 46 via the separate gateway 60 (e.g., utilizing highspeed low latency networks). Sources of patient vital data 40 are connected to the anesthesia system 12. The anesthesia system 12, the ultrasound imaging device 54, the edge gateway 60, and the cloud-based storage/analysis system 46 are as described in FIG. 4. However, a first highspeed low latency network 62 is provided between the gateway 60 and the anesthesia system 12 and a second highspeed low latency network 64 is provided between the gateway 60 and the ultrasound imaging device 54. The highspeed low latency networks 62, 64 ensure a more real time synchronization to help aid even remote procedures. For example, robot may be used for the surgery and holding ultrasound probe while being controlled by a clinician far away from the location of the surgery. In this scenario, no mission critical application or data depend on the cloud-based storage/analysis system 46. Mission critical data does not include the analytics data. However, the mission critical data is utilized to generate the analytics without affecting the completion of the critical operation.

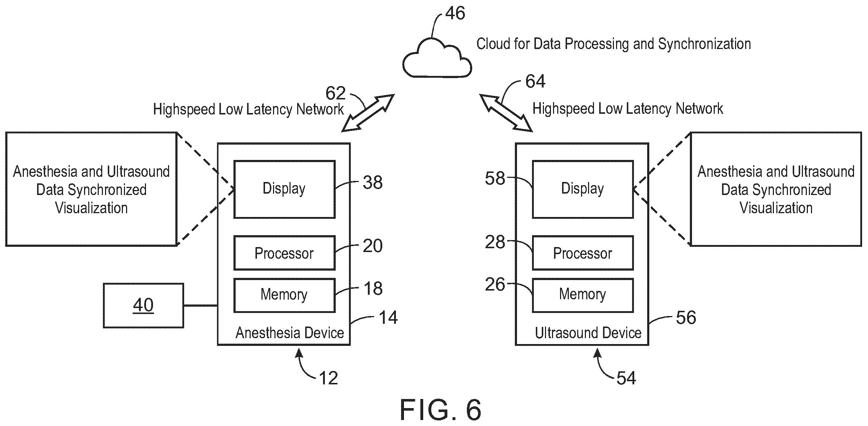

FIG. 6 is a schematic diagram of the separate anesthesia system 12 and the separate ultrasound imaging device 54 communicating directly with the cloud-based storage/analysis system 46 (e.g., utilizing highspeed low latency networks). Sources of patient vital data 40 are connected to the anesthesia system 12. The anesthesia system 12, the ultrasound imaging device 54, and the cloud-based storage/analysis system 46 are as described in FIG. 4. However, the first highspeed low latency network 62 is provided directly between the cloud-based storage/analysis system 46 and the anesthesia system 12 and the second highspeed low latency network 64 is provided directly between the cloud-based storage/analysis system 46 and the ultrasound imaging device 54. Thus, a gateway is not utilized between the highspeed low latency networks 62, 64 and the cloud-based storage/analysis system 46. The highspeed low latency networks 62, 64 ensure a more real time synchronization to help aid even remote procedures. For example, robot may be used for the surgery and holding ultrasound probe while being controlled by a clinician far away from the location of the surgery. In this scenario, no mission critical application or data depend on the cloud-based storage/analysis system 46. Mission critical data does not include the analytics data. However, the mission critical data is utilized to generate the analytics without affecting the completion of the critical operation.

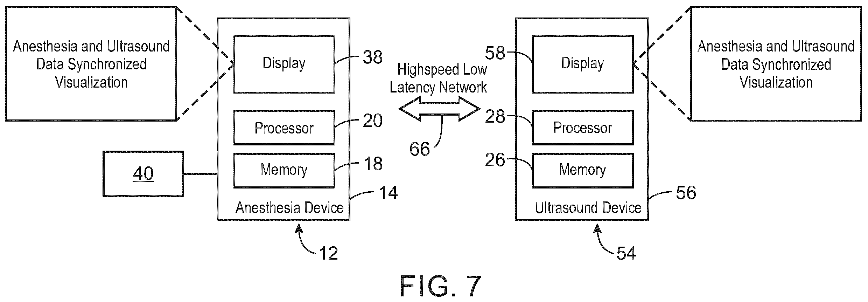

FIG. 7 is a schematic diagram of the separate anesthesia system 12 and the separate ultrasound imaging device 54 communicating directly with each other (e.g., utilizing a highspeed low latency network 66). Sources of patient vital data 40 are connected to the anesthesia system 12. The anesthesia system 12 and the ultrasound imaging device 54 are as described in FIG. 4. However, the highspeed low latency network 66 is provided directly between the anesthesia system 12 and the ultrasound imaging device 54. Thus, a cloud-based system is not utilized. The highspeed low latency networks 66 ensure a more real time synchronization to help aid even remote procedures. For example, robot may be used for the surgery and holding ultrasound probe while being controlled by a clinician far away from the location of the surgery. In this scenario, no mission critical application or data depend on the cloud-based storage/analysis system 46. Mission critical data does not include the analytics data. However, the mission critical data is utilized to generate the analytics without affecting the completion of the critical operation.

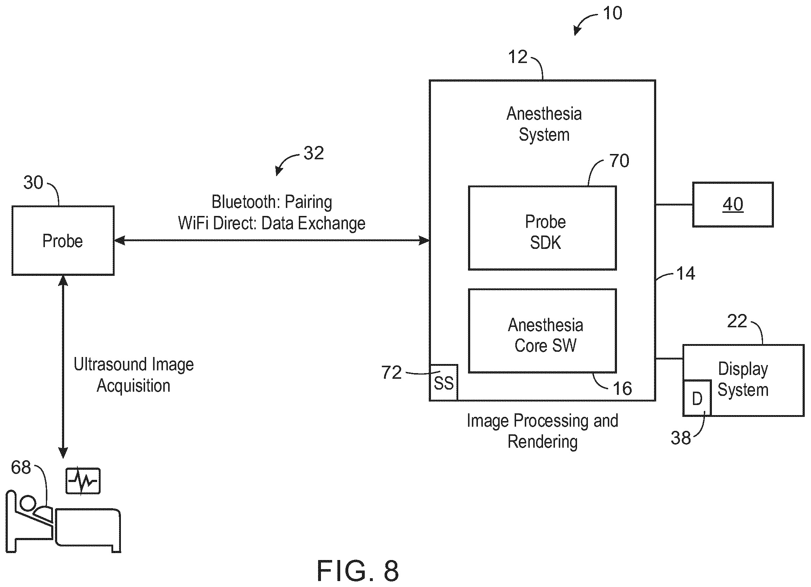

FIG. 8 is a schematic diagram of the ultrasound probe 30 (e.g., wireless ultrasound probe such as Vscan Air^{™} from GE Healthcare) communicatively coupled via a wireless connection to the anesthesia system 12. Sources of patient vital data 40 are connected to the anesthesia system 12. The anesthesia system 12 is part of the integrated system 10 as describe in FIG. 8. As depicted, the ultrasound probe 30 is utilized to perform an ultrasound image acquisition on a patient 68 (e.g., subjected to anesthesia therapy and being monitored via the anesthesia system 12). In certain embodiments, the ultrasound probe 30 is wirelessly (e.g., via Bluetooth^{®}) with the anesthesia system 12. In certain embodiments, the ultrasound probe 30 exchanges data bidirectionally (e.g., via Wi-Fi Direct) with the anesthesia monitoring system 12. A probe software development kit (SDK) 70 (e.g., display system software) (e.g., as part of the ultrasound core 24 in FIG. 1) is disposed within the housing 14 of the anesthesia system 12. The probe SDK 70 is configured to process the scan data acquired by the ultrasound probe 30 and to provide valuable imaging information to the clinician. The anesthesia system 12 is configured to handle the additional processing and communication requirements of the ultrasound imaging system 32.

The display system 22 of the anesthesia system 12 is configured to act as a user interface for the ultrasound imaging system 32. In particular, the probe SDK 70 is configured to display the ultrasound imaging, anesthesia therapy settings and the anesthesia monitoring parameters side by side on the same display 38 or separate displays 38 of the display system 22. The time stamping for the patient anesthesia therapy settings, anesthesia monitoring parameters with the ultrasound imaging data occurs on the anesthesia system 12 (e.g., by the anesthesia core 16 and/or the probe SDK 70). By request of the clinician the data can be overlapped and shown on the same display 38. In certain embodiments, when dual displays 38 are utilized by the anesthesia system 12, ultrasound imaging can be seen on a first display 38 and the anesthesia therapy settings and patient anesthesia monitoring parameters can be seen on the second display 38 or vice versa depending on the preferences of the anesthesiologist and/or clinician. In certain embodiments, the ultrasound probe 30 can be stored in a storage space (e.g., storage box) on the housing 14 or disposed within the housing 14 of the anesthesia system 12. In certain embodiments, the ultrasound probe 30 can be stored outside of and separate from the anesthesia system 12. In certain embodiments, the anesthesia system 12 has one of the displays 38 (e.g., a tablet display) coupled to a mechanical or robotic arm mounted on a top or on a side the anesthesia system 12 that enables the display 38 to be moved closer to the clinician performing the ultrasound imaging.

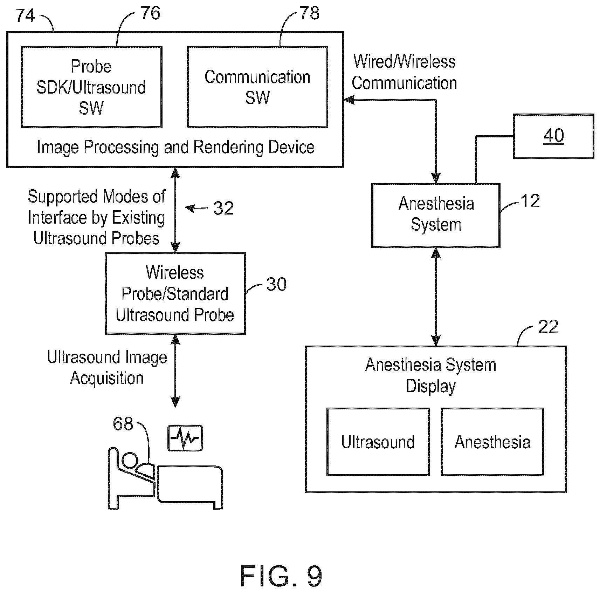

FIG. 9 is a schematic diagram of the ultrasound probe 30 communicatively coupled to a separate device 74 communicatively coupled to the anesthesia system 12. Sources of patient vital data 40 are connected to the anesthesia system 12. The device 74 and the ultrasound probe 30 form the ultrasound imaging system 32. As depicted, the ultrasound probe 30 is utilized to perform an ultrasound image acquisition on a patient 68 (e.g., subjected to anesthesia therapy and being monitored via the anesthesia system 12). In certain embodiments, the ultrasound probe 30 (e.g., wireless ultrasound probe such as Vscan Air^{™} from GE Healthcare) is communicatively coupled to the device 74 via a wireless connection. In certain embodiments, the ultrasound probe 30 is communicatively coupled to the device 74 via a wired connection. The device 74 includes probe SDK/ultrasound software (SW) 76 (and hardware) and communication software 78. The probe SDK/ultrasound software 76 is configured to perform image processing and rendering on the scan data received from the ultrasound probe 30. The device 74 is configured to be communicatively coupled to the anesthesia system 12. In certain embodiments, the device 74 is communicatively coupled to the anesthesia system 12 via a wireless connection. In certain embodiments, the device 74 is communicatively coupled to the anesthesia system 12 via a wired connection.

The display system 22 of the anesthesia system 12 is configured to act as a user interface for the ultrasound imaging system 32. In particular, the anesthesia system 12 is configured to display the ultrasound imaging, anesthesia therapy settings and the anesthesia monitoring parameters side by side on the same display or separate displays of the display system 22. The time stamping for the patient anesthesia therapy settings and anesthesia monitoring parameters with the ultrasound imaging data occurs on the anesthesia system 12. By request of the clinician data can be overlapped and shown on the same display of the display system 22. In certain embodiments, when dual displays are utilized by the anesthesia system 12, ultrasound imaging can be seen on a first display and the anesthesia therapy settings and patient anesthesia monitoring parameters can be seen on the second display or vice versa depending on the preferences of the anesthesiologist and/or clinician.

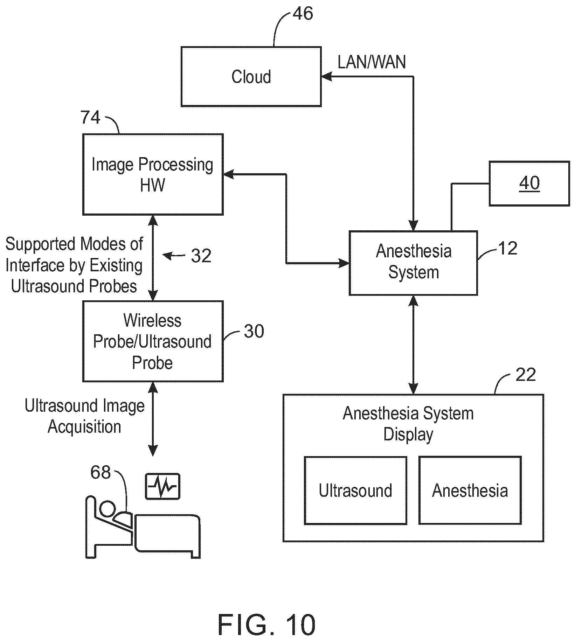

FIG. 10 is a schematic diagram of the ultrasound probe 30 communicatively coupled to the separate device 74 communicatively coupled to the anesthesia system 12 (which is communicatively coupled to the cloud-based storage/analysis system 46). Sources of patient vital data 40 are connected to the anesthesia system 12. The device 74 and the ultrasound probe 30 form the ultrasound imaging system 32. As depicted, the ultrasound probe 30 is utilized to perform an ultrasound image acquisition on a patient 68 (e.g., subjected to anesthesia therapy and being monitored via the anesthesia system 12). In certain embodiments, the ultrasound probe 30 (e.g., wireless ultrasound probe such as Vscan Air^{™} from GE Healthcare) is communicatively coupled to the device 74 via a wireless connection. In certain embodiments, the ultrasound probe 30 is communicatively coupled to the device 74 via a wired connection. The device 74 includes software and hardware to perform image processing and rendering on the scan data received from the ultrasound probe 30. The device 74 is configured to be communicatively coupled to the anesthesia system 12. In certain embodiments, the device 74 is communicatively coupled to the anesthesia system 12 via a wireless connection. In certain embodiments, the device 74 is communicatively coupled to the anesthesia system 12 via a wired connection. The anesthesia system 12 is configured to act as a gateway for providing the ultrasound imaging data, anesthesia therapy settings and anesthesia monitoring parameters data to the cloud-based storage/analysis system 46. In certain embodiments, the cloud-based storage/analysis system 46 is configured to analyze and to synchronize (e.g., add time stamps) the patient vital data and the ultrasound data. The synchronized data and meaningful insights from the analysis is relayed from the cloud-based storage/analysis system 46 back to both the anesthesia system 12 and/or the device 74. In certain embodiments, the ultrasound data, anesthesia therapy settings and the anesthesia monitoring parameters data may be stored on the cloud-based storage/analysis system 46. In certain embodiments, the ultrasound data, anesthesia therapy settings and the anesthesia monitoring parameters data may be stored on the anesthesia system 12.

The display system 22 of the anesthesia system 12 is configured to act as a user interface for the ultrasound imaging system 32. In certain embodiments, the display system 22 may act as the primary display for the ultrasound imaging. In certain embodiments, the display system 22 may act as the secondary display for the ultrasound imaging (e.g., the primary display may be associated with another device communicatively coupled to the cloud-based storage/analysis system 46). In particular, the anesthesia system 12 is configured to display the ultrasound imaging, anesthesia therapy settings and the anesthesia monitoring parameters side by side on the same display or separate displays of the display system 22. The time stamping for the patient anesthesia therapy settings, anesthesia monitoring parameters with the ultrasound imaging data occurs on the anesthesia system 12. By request of the clinician the data can be overlapped and shown on the same display of the display system 22. In certain embodiments, when dual displays are utilized by the anesthesia system 12, ultrasound imaging can be seen on a first display and the anesthesia therapy settings and patient anesthesia monitoring parameters can be seen on the second display or vice versa depending on the preferences of the anesthesiologist and/or clinician.

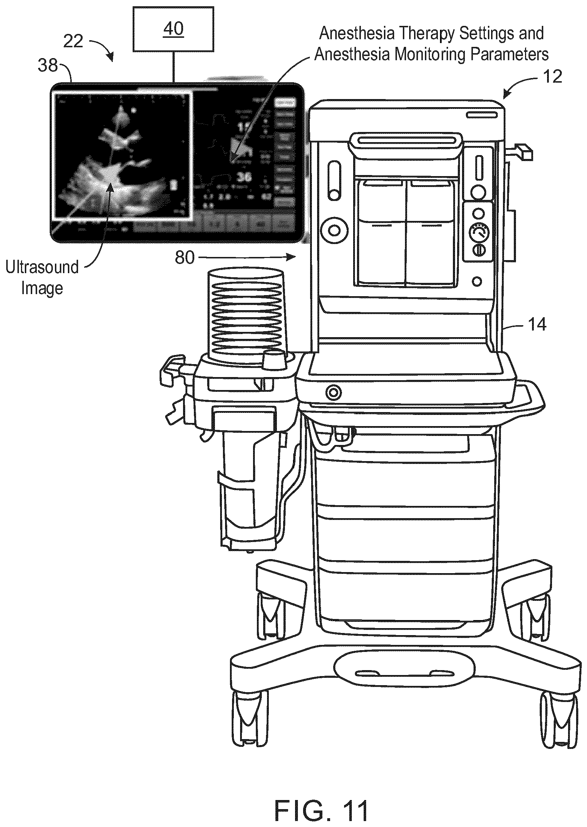

FIG. 11 is a schematic diagram of the anesthesia system 12 configured to display ultrasound data, anesthesia therapy settings and anesthesia monitoring parameter data (e.g., having a single display 38). Sources of patient vital data 40 are connected to the anesthesia system 12. In certain embodiments, the anesthesia monitoring system 12 is the integrated system 10 described in FIG. 1. As depicted, the display system 22 includes a single display 38. As depicted, the display 38 is coupled to the housing 14 of the anesthesia system 12. In particular, as depicted, the display 38 is coupled to one side 80 of the housing 14. In certain embodiments, the display 38 may be coupled to the opposite side of the housing 14. In certain embodiments, the display 38 may be coupled to a top of the housing 14. As depicted, the display 38 is simultaneously displaying an ultrasound image, anesthesia therapy settings and anesthesia monitoring parameters (e.g., measured parameters of the patient, heart waveforms, etc.). In certain embodiments, the ultrasound image may also include measurements. In certain embodiments, anesthesia settings (e.g., for applying the anesthesia therapy) may be provided simultaneously with the ultrasound image and/or anesthesia monitoring parameters. In certain embodiments, the display 38 may be configured to receive user input to switch to one of the ultrasound image, the anesthesia monitoring parameters, or anesthesia therapy setting for sole display, or to two of the ultrasound image, the anesthesia monitoring parameters, or anesthesia therapy setting for simultaneous display, or each of the ultrasound image, the anesthesia monitoring parameters, and anesthesia therapy setting for simultaneous display. In certain embodiments, the display 38 may be configured to receive user input to change the size of one of the ultrasound image, the anesthesia monitoring parameters, and anesthesia therapy setting relative to the others.

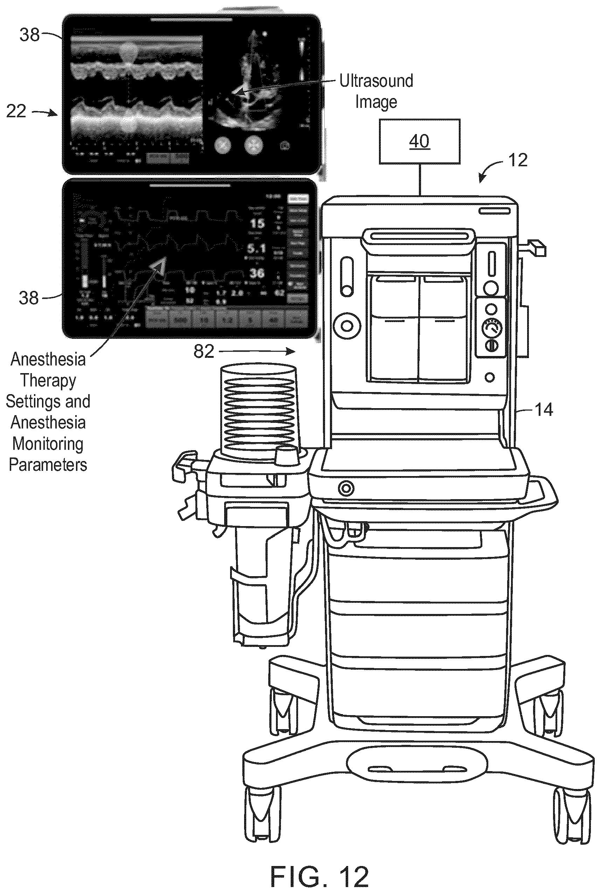

FIG. 12 is a schematic diagram of the anesthesia system 12 configured to display ultrasound data, anesthesia therapy settings and anesthesia monitoring parameters data (e.g., having a pair of displays 38). Sources of patient vital data 40 are connected to the anesthesia system 12. In certain embodiments, the anesthesia system 12 is the integrated system 10 described in FIG. 1. As depicted, the display system 22 includes a pair of displays 38. As depicted, the pair of displays 38 is coupled to the housing 14 of the anesthesia system 12. In particular, as depicted, the pair of displays 38 is coupled to one side 82 of the housing 14. In certain embodiments, the pair of displays 38 may be coupled to the opposite side of the housing 14. In certain embodiments, the pair of displays 38 may be coupled to a top of the housing 14. As depicted, the pairs of displays 38 are arranged with one of the displays 38 located above the other display 38. As depicted, one display 38 (i.e., the top display 38) is displaying the ultrasound image, while the other display 38 (i.e., the bottom display 38) is displaying the anesthesia monitoring parameters (e.g., measured parameters of the patient, heart waveforms, etc.). In certain embodiments, the ultrasound image may also include measurements. In certain embodiments, anesthesia settings (e.g., for applying the anesthesia therapy) may be displayed on the one of the displays 38. For example, two of the ultrasound image, the anesthesia monitoring parameters, and the anesthesia therapy settings may be displayed on one display 38 while the remaining item is displayed on the other display 38. In certain embodiments, each of the ultrasound image, the anesthesia monitoring parameters, and the anesthesia therapy settings may be simultaneously displayed on one display 38, while entertainment (e.g., movies, videos, music, audio recordings, etc.) may be displayed on the other display 38. In certain embodiments, one or both of the displays 38 may be configured to receive user input (e.g., user interface on the displays 38 or a separate user interface or user input device such as a button, switch, keyboard, etc.) to switch to one of the ultrasound image, the anesthesia monitoring parameters and anesthesia therapy setting for sole display, or to two of the ultrasound image, the anesthesia monitoring parameters, or anesthesia therapy setting for simultaneous display, or each of the ultrasound image, the anesthesia monitoring parameters and anesthesia therapy setting for simultaneous display. In certain embodiments, one or both of the displays 38 may be configured to receive user input (e.g., user interface on the displays 38 or a separate user interface or user input device such as a button, switch, keyboard, etc.) to change the size of one of the ultrasound image, the anesthesia monitoring parameters and anesthesia therapy setting relative to the others.

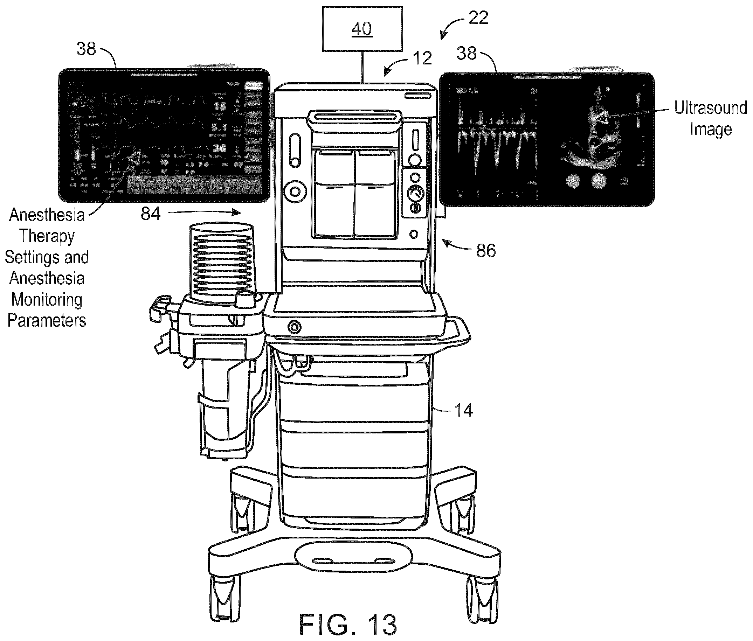

FIG. 13 is a schematic diagram of the anesthesia system 12 configured to display ultrasound data and anesthesia data (e.g., also having a pair of displays 38 in a different arrangement). Sources of patient vital data 40 are connected to the anesthesia system 12. In certain embodiments, the anesthesia system 12 is the integrated system 10 described in FIG. 1. As depicted, the display system 22 includes a pair of displays 38. As depicted, the pair of displays 38 is coupled to the housing 14 of the anesthesia system 12. Sources of patient vital data 40 are also coupled to the anesthesia system 12. In particular, as depicted, the displays 38 are coupled to opposite sides 84, 86 of the housing 14. As depicted, one display 38 (i.e., the right display 38) is displaying the ultrasound image, while the other display 38 (i.e., the left display 38) is displaying the anesthesia monitoring parameters (e.g., measured parameters of the patient, heart waveforms, etc.). In certain embodiments, the ultrasound image may also include measurements. In certain embodiments, anesthesia settings (e.g., for applying the anesthesia therapy) may be displayed on the one of the displays 38. For example, one ultrasound image, the anesthesia monitoring parameters and the anesthesia therapy settings may be displayed on one display 38 while the remaining item is displayed on the other display 38. In certain embodiments, each of the ultrasound image, the anesthesia monitoring parameters and the anesthesia therapy settings may be simultaneously displayed on one display 38, while entertainment (e.g., movies, videos, music, audio recordings, etc.) may be displayed on the other display 38. In certain embodiments, one or both of the displays 38 may be configured to receive user input (e.g., user interface on the displays 38 or a separate user interface or user input device such as a button, switch, keyboard, etc.) to switch to one of the ultrasound image, the anesthesia monitoring parameters, or anesthesia therapy setting for sole display, or to two of the ultrasound images, the anesthesia monitoring parameters, or anesthesia therapy setting for simultaneous display, or each of the ultrasound image, the anesthesia monitoring parameters and anesthesia therapy setting for simultaneous display. In certain embodiments, one or both of the displays 38 may be configured to receive user input (e.g., user interface on the displays 38 or a separate user interface or user input device such as a button, switch, keyboard, etc.) to change the size of one of the ultrasound images, the anesthesia monitoring parameters, and anesthesia therapy setting relative to the others.

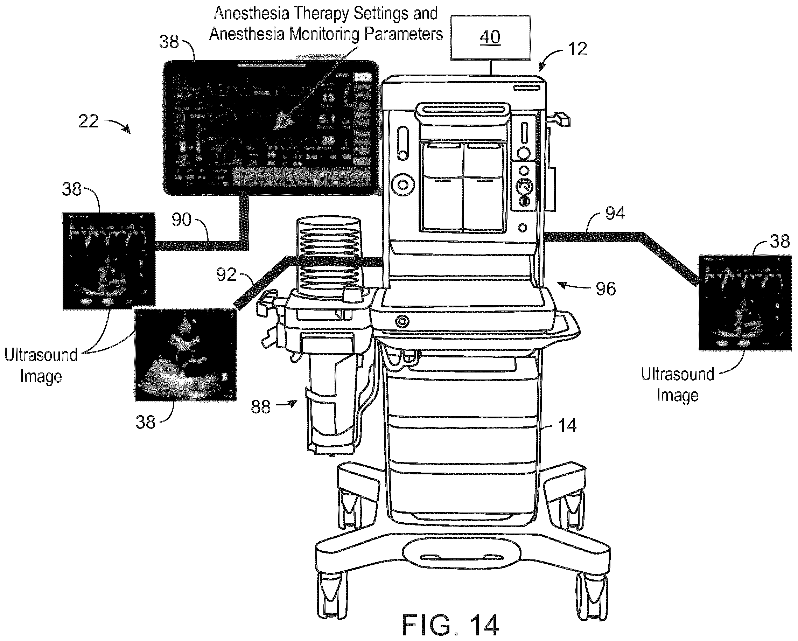

FIG. 14 is a schematic diagram of the anesthesia system 12 configured to display ultrasound data and anesthesia data (e.g., having a plurality of displays). In certain embodiments, the anesthesia system 12 is the integrated system 10 described in FIG. 1. As depicted, the display system 22 includes four displays 38 (i.e., a larger display 38 and three smaller displays 38). The size of the displays 38 and the size of the displays 38 relative to each other may vary. As depicted, the larger display 38 is coupled to the housing 14 of the anesthesia monitoring system 12. Sources of patient vital data 40 are also coupled to the anesthesia system 12. In particular, the larger display 38 is coupled directly to a side 88 of the housing 14. Also, a smaller display 38 is coupled via a robotic or mechanical arm 90 to the larger display 38. Another smaller display 38 is coupled via a robotic or mechanical arm 92 to the side 88 of the housing 14. A further smaller display 38 is coupled via a robotic or mechanical arm 94 to an opposite side 96 of the housing 14. The robotic or mechanical arms 90, 92, and 94 enable the user to move the displays 38 to a desired location.

As depicted, one display 38 (i.e., the upper display 38) is displaying the anesthesia parameters (e.g., measured parameters of the patient, heart waveforms, etc.), while the other displays 38 (i.e., the smaller displays 38) are displaying ultrasound images. In certain embodiments, the ultrasound images may also include measurements. In certain embodiments, anesthesia settings (e.g., for applying the anesthesia therapy) may be displayed on the one of the displays 38. A combinations of the ultrasound images, the anesthesia monitoring parameters, and/or the anesthesia therapy settings may be displayed on the displays. In certain embodiments, each of the ultrasound images, the anesthesia monitoring parameters and the anesthesia therapy settings may be simultaneously displayed on one display 38, while entertainment (e.g., movies, videos, music, audio recordings, etc.) may be displayed on one of the other displays 38. In certain embodiments, one or more of the displays 38 may be configured to receive user input (e.g., user interface on the displays 38 or a separate user interface or user input device such as a button, switch, keyboard, etc.) to switch to one of the ultrasound images, the anesthesia parameters, or anesthesia setting for sole display, or to two of the ultrasound images, the anesthesia monitoring parameters, or anesthesia therapy setting for simultaneously display, or each of the ultrasound images, the anesthesia monitoring parameters, and anesthesia therapy setting for simultaneous display. In certain embodiments, one or more of the displays 38 may be configured to receive user input (e.g., user interface on the displays 38 or a separate user interface or user input device such as a button, switch, keyboard, etc.) to change the size of one of the ultrasound images, the anesthesia monitoring parameters and anesthesia therapy setting relative to the others.

Technical effects of the disclosed embodiments include enabling more effective use of operating room space. Technical effects of the disclosed embodiments also include reducing the stress on the clinician and anesthesiologist by providing ultrasound data, anesthesia therapy settings and anesthesia monitoring parameters data on a single display. The technical effects of the disclosed embodiments further include providing ultrasound imaging capability in an anesthesia system will aid the anesthesiologist in making more accurate predictions and produce better outcomes for the patient. The technical effects of the disclosed embodiments include making the machine (e.g., anesthesia system) safer, more accurate, and faster. The technical effects of the disclosed embodiments include eliminating the form factor of the equipment used for ultrasound imaging

The techniques presented and claimed herein are referenced and applied to material objects and concrete examples of a practical nature that demonstrably improve the present technical field and, as such, are not abstract, intangible or purely theoretical. Further, if any claims appended to the end of this specification contain one or more elements designated as "means for [perform]ing [a function]..." or "step for [perform]ing [a function]...", it is intended that such elements are to be interpreted under 35 U.S.C. 112(f). However, for any claims containing elements designated in any other manner, it is intended that such elements are not to be interpreted under 35 U.S.C. 112(f).

This written description uses examples to disclose the present subject matter, including the best mode, and also to enable any person skilled in the art to practice the invention, including making and using any devices or systems and performing any incorporated methods. The patentable scope of the subject matter is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. An anesthesia system, comprising:
a housing;
a display system coupled to housing;
an ultrasound probe communicatively coupled to the anesthesia system;
a memory encoding processor-executable routines disposed within the housing; and
a processing system disposed within the housing, wherein the processing system comprises one or more processors and is configured to access the memory and to execute the processor-executable routines, wherein the processor-executable routines, when executed by the processing system, cause the processing system to:
receive scan data of a subject receiving anesthesia therapy from the ultrasound probe;
receive patient vital data representative of one or more anesthesia monitoring parameters of the subject from sources of patient vital data or sensors; and
display the one or more anesthesia monitoring parameters and an ultrasound image derived from the scan data on the display system.

2. The anesthesia system of claim 1, wherein the display system comprises a display and the one or more anesthesia monitoring parameters and the ultrasound image are simultaneously displayed on the display.

3. The anesthesia system of claim 1, wherein the display system comprises a plurality of displays, and the one or more anesthesia monitoring parameters and the ultrasound image are simultaneously displayed on separate displays of the plurality of displays.

4. The anesthesia system of claim 1, wherein the processor-executable routines, when executed by the processing system, cause the processing system to display anesthesia therapy settings on the display system.

5. The anesthesia system of claim 4, wherein the display system comprises a display and the one or more anesthesia monitoring parameters, the ultrasound image, and the anesthesia therapy settings are simultaneously displayed on the display.

6. The anesthesia system of claim 1, wherein the one or more anesthesia monitoring parameters and the ultrasound image are displayed in real time on the display system.

7. The anesthesia system of claim 1, wherein the processor-executable routines, when executed by the processing system, cause the processing system to store the scan data and the patient vital data in the memory.

8. The anesthesia system of claim 1, wherein the processor-executable routines, when executed by the processing system, cause the processing system to:
provide the scan data and the patient vital data to a cloud-based system for processing and analysis; and
receive the ultrasound image, the one or more anesthesia monitoring parameters, and the analysis of the scan data and the patient vital data from the cloud-based system.

9. The anesthesia system of claim 8, wherein the processor-executable routines, when executed by the processing system, cause the processing system to provide the scan data and the patient vital data to a cloud-based system for storage.

10. The anesthesia system of claim 1, wherein the processor-executable routines, when executed by the processing system, cause the processing system to provide the scan data and the patient vital data to a cloud-based system for synchronization of the data prior to display of the one or more anesthesia monitoring parameters and the ultrasound image.

11. The anesthesia system of claim 1, wherein the processor-executable routines, when executed by the processing system, cause the processing system to synchronize the scan data and the patient vital data.

12. The anesthesia system of claim 1, processor-executable routines, when executed by the processing system, cause the processing system to provide the scan data and the patient vital data to one or more of a hospital network, a picture archiving and communication system, an electronics medical records system, or a cloud-based storage/analytics and insights system.

13. The anesthesia system of claim 1, wherein the ultrasound probe is communicatively coupled to the anesthesia system via a wired connection.

14. The anesthesia system of claim 1, wherein the ultrasound probe is communicatively coupled to the anesthesia system via a wireless connection.
